# EUROPEAN PATENT APPLICATION

(11) **EP 4 454 623 A1**
(43) Date of publication of application: **30.10.2024**
(21) Application number: 21824013.3
(22) Date of filing: 15.10.2021
(51) Int. Cl.: A61F 13/06, A41B 11/00, A41D 13/05, A41D 13/12

(54) **ACTIVE CLOTHING FOR THE PREVENTION OF PRESSURE ULCERS**

(30) Priority: 14.10.2021 PT 2021117517
(71) Applicant: Escola Superior de Enfermagem de Coimbra, 3001-901 Coimbra (PT); Impetus Portugal - Têxteis S.A., 4740-696 Barqueiros (PT); Universidade do Minho, 4704-553 Braga (PT)
(72) Inventor: DINIS PARREIRA, Pedro Miguel, Coimbra, 3045-293 Ameal (PT); SILVA, Christiana, 3001-901 Coimbra (PT); RAMOS, Aurélie, 3001-901 Coimbra (PT); DE SOUSA SALGUEIRO OLIVEIRA, Anabela, 3030-175 Coimbra (PT); MONTEZUMA CARVALHO MENDES VAQUINHAS, Marina, 3030-320 Coimbra (PT); ALVES APÓSTOLO, João Luis, 3045-120 Coimbra (PT); BERNARDES, Rafael, 3000-187 Coimbra (PT); BAPTISTA SOUSA, Liliana, 3140-305 Pereira (PT); LOPES PARREIRA, Joana Íris, 3045-293 Coimbra (PT); CONTENTE RANGEL HENRIQUES, Mariana, 4705-630 Braga (PT); ESTEVES DE SOUSA FANGUEIRO, Raul Manuel, 4700-150 Braga (PT); FERNANDES CARVALHO, Migual Ângelo, 4700-384 Braga (PT); MARIZ FIGUEIREDO, Ricardo Alberto, 4740-141 Apúlia (PT); CORREIA DA CRUZ, Juliana Patrícia, 4765-220 Famalicão (PT)
(74) Representative: Patentree
(86) International application number: PCT/IB2021/059523
(87) International publication number: WO 2023/062419

(57) **Abstract**

The present invention relates to clothing comprising a material designed to alleviate the pressure in places corresponding to bony prominences, thereby promoting greater comfort and, implicitly, enhancing the person's privacy due to its capacity to improve the quality of life by relieving the pressure in the bony prominences which are more susceptible to the development of pressure ulcers, thus contributing to maintain the skin resistance and improving the microclimate in the area, in order to reduce the risk of development of such ulcers. The present invention is therefore directed at lowering the occurrence of pressure ulcers among people with impaired physical mobility. For example, a pair of socks (5) or a pyjama, wherein the material (2), preferably a polymeric material with active substances, is arranged so as to protect said bony prominences, namely the malleoli and calcaneus.

## Description

### SCOPE AND BACKGROUND OF THE INVENTION

The world society is currently facing the problem of demographic ageing. The increasing ageing of the population and the growing number of people suffering from partial or total inability make them more prone to developing health conditions. Therefore, and despite advances in healthcare services, pressure ulcers remain an important cause of morbidity and mortality.

A pressure ulcer is a localised injury to the skin and/or underlying tissue, usually over a bony prominence, resulting from pressure or a combination of the latter with shear, being also defined as *"localised areas of the body surface that have suffered prolonged exposure to high pressure, friction or stretching, thereby hindering the local blood flow, with consequent tissue destruction and*/*or necrosis".*

There is a predominance of pressure ulcers in the sacroccygeal, sacrococcyx and calcaneal regions, as well as in the sacrococcygeal, trochanter, malleolar, buttock and heel regions. Thus, the following are some preferential areas for the emergence of pressure ulcers: sacrococcygeal region, trochanter/ iliac crest, scapular region, olecranon, calcaneus and malleoli (DGS - Portuguese Directorate-General for Health, 2007).

Pressure-relief strategies are crucial for the prevention as well as the treatment of pressure ulcers. Pressure relief of tissues presupposes the avoidance of anoxia, tissue ischaemia and pressure-related injuries, including infections, thus increasing soft tissues viability and providing the lesion (in case of already existing pressure ulcers) optimal conditions for healing. This pressure relief may be achieved by using positioning techniques and by choosing an adequate support surface (GNEAUPP, 2003). Various pressure-reducing devices are currently used in order to redistribute the person's body weight over a larger surface area, such as alternating pressure mattresses, pillows, elbow pads, heel pads, and other supports made up of foam and gel (DGS - Directorate-General for Health, 2007). In addition to pressure relief measures, incontinence control, skin care and nutrition are also recommended (*Luz et al.,* 2010) . A careful examination of the patient is obviously essential to better adapt the preventive measures to be taken.

### SUMMARY OF THE INVENTION

The object of the present invention is, therefore, clothing for the prevention of pressure ulcers, which comprises polymeric materials with active substances in the places corresponding to the bony prominences, in order to relieve/reduce said pressure. We know that the body weight at rest on bony prominences (scapula, olecranon, dorsal region, iliac crest, trochanter, sacrocoxygeal region, among others) originates significant concentrations of pressure on the skin surface, causing poor nutrition and lack of oxygenation of the tissues.

The present invention is useful as it enables healthcare professionals, or even informal caregivers, to better streamlining the resources and improving time management in the prevention of pressure ulcers. The safe use of this clothing does not require clients to have prior knowledge regarding the pressure ulcers and body regions most vulnerable to their development. Thus, informal caregivers as well as the individuals themselves can make use of this clothing.

Namely, this invention may present the materials arranged in strategic places of pressure concentration or incorporate devices with sensing/monitoring features as regards critical parameters (precursors of/related to) pressure ulcer development, such as pressure, moisture, temperature, tangential/cutting forces/stresses. The functionalities are thus presented through encapsulated active agents.

### DESCRIPTION OF THE FIGURES

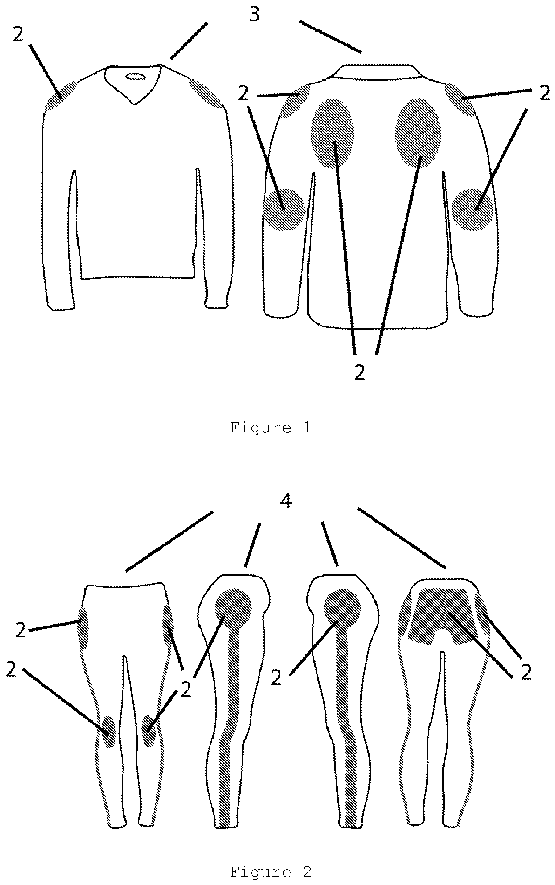

Figure 1 - representation of an embodiment corresponding to a shirt/jacket (3), comprising preferably polymeric-based material (2), with release of active substances, strategically arranged in places corresponding to the scapula, olecranon and dorsal region, thus protecting the said scapula, olecranon and dorsal regions, these being illustrated as the spots filled with shades of blue.
Figure 2 - representation of an embodiment corresponding to trousers (4), comprising preferably polymeric-based material (2), with release of active substances, which is arranged to protect the iliac crest, trochanter, sacrococcygeal region, line of the external region of the lower limbs and between the knees, these areas being illustrated as the spots filled with shades of blue.

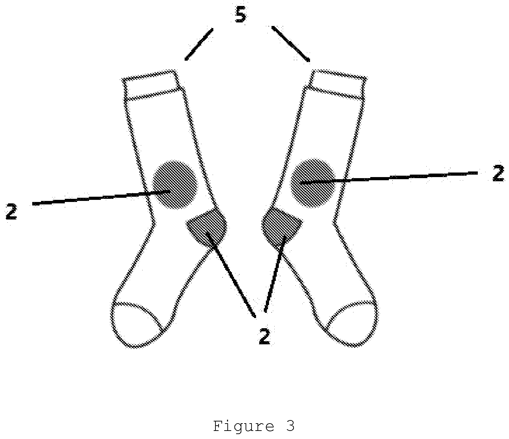

Figure 3 - representation of an embodiment corresponding to a pair of socks (5) in which a preferably polymeric-based material (2), with release of active substances, is arranged so as to protect the malleoli and calcaneus, these areas being illustrated as the spots filled with shades of grey.

### DETAILED DESCRIPTION OF THE INVENTION

The present invention relates to clothing (1) comprising a material (2) designed to alleviate the pressure in places corresponding to bony prominences, promoting greater comfort and, implicitly, enhancing the patient privacy due to its capacity to improve the quality of life by relieving the pressure in the bony prominences which are more susceptible to the development of pressure ulcers, thus contributing to keep the integrity of the skin and reducing the risk of appearance of said ulcers, as well as related infections.

In a preferred embodiment, the material (2) which alleviates the pressure in places corresponding to bony prominences is a polymeric-based material with release of active substances. The polymeric-based protection systems can be incorporated into the clothing directly on the application, by means of coating, stamping and/or localised lining techniques with thermally expandable polymeric materials, such as polyurethane-based polymers, EVA, Newtonian or non-Newtonian polymeric fluids and the like, for the purpose of absorbing impact energy and thus increasing the contact area. This energy absorption system will be coupled, in the form of pockets or lamination to cellulose-based knit structures, such as cotton, modal and its derivatives, functionalized with natural active substances, based on essential oils. These active substances can functionalize the textiles by means of two systems: one is microencapsulation where there is an active release of the substances until depleted; the second one is coupling to the textile substrate a refillable system of active substances during washes, through functionalization of the substrates with a system like porous microcapsules or cyclodextrins.

This system is fully washable, with the possibility to recharge the active functionalization during the washing process, and it can be used and handled as any other clothing.

In a preferred embodiment, the invention is a shirt/jacket (3) comprising the material (2), preferably a polymeric material with natural active substances, strategically arranged in places corresponding to the scapula, olecranon and dorsal region, thereby protecting the body in the scapula, olecranon and dorsal regions.

In another preferred embodiment, the invention, is a trouser (4) in which the material (2), preferably polymeric material with natural active substances, is arranged in order to protect the iliac crest, trochanter, sacrococcygeal region, line of the external region of the lower limbs and between the knees.

In another preferred embodiment, the invention is a pair of socks (5) in which the material (2), preferably a polymeric material with natural active substances, is arranged in order to protect the malleoli and calcaneus.

In another preferred embodiment of the present invention, the materials arranged in strategic places of pressure concentration have, or incorporate, devices with sensing/monitoring features as regards critical parameters (precursors of/related to) pressure ulcer development, such as pressure, moisture, temperature, tangential/cutting forces/stresses.

In another preferred embodiment, the latter may comprise a combination of the shirt/jacket (3), trousers (4) and pair of socks (5), functioning as a pyjama, preferably in natural and soft touch textiles, such as cotton, modal, lyocell and blends with capillary fibres such as biodegradable polyamide or PLA, with polymeric materials comprising active substances, coupled at the locations corresponding to the body's bone prominences, in each of the preferred embodiments.

As will be evident to a person skilled in the art, the present invention should not be limited to the embodiments described herein, with a number of changes being possible, which remain within the scope of this invention.

Of course, the preferred embodiments above described are combinable in the different possible forms, the repetition of all such combinations being herein avoided.

## Claims

1. Active clothing for the prevention of pressure ulcers (1), comprising a polymer-based material (2) with release of active substances, said material (2) being arranged on the corresponding bony prominences and alleviating the pressure at those places.

2. Clothing according to the previous claim, wherein the material (2) is directly applied to the clothing by coating, stamping and/or localised lining techniques with thermally expandable polymeric materials such as a polyurethane-based polymer, EVA, Newtonian or non-Newtonian polymeric fluids and the like.

3. Clothing according to claim 1, wherein the material (2) is coupled, in the form of pockets or lamination, to cellulose-based knit structures, such as cotton, modal and its derivatives, functionalized with natural active substances, based on essential oils.

4. Clothing according to the preceding claims, wherein the active substances are microencapsulated and released until depleted; and/or coupled to the textile substrate, the active substances being refillable during washing, by functionalizing the substrates with a system of porous microcapsules or cyclodextrins.

5. Clothing according to the preceding claims, wherein said clothing is a shirt/jacket (3) comprising the material (2) arranged at places corresponding to the scapula, olecranon and dorsal region, thereby protecting the body in the scapula, olecranon and dorsal regions.

6. Clothing according to the claims 1 to 4, wherein said clothing is a trouser (4) in which the material (2) is arranged to protect the iliac crest, trochanter, sacrococcygeal region, line of the external region of the lower limbs and between the knees.

7. Clothing according to the claims 1 to 4, wherein said clothing is a pair of socks (5) in which the material (2) is arranged so as to protect the malleoli and heels.

8. Clothing according to the preceding claims, wherein the materials arranged at strategic locations of pressure concentration have, and/or incorporate, sensing/monitoring devices.

9. Clothing according to the preceding claims, wherein said clothing is a combination of shirt/jacket (3), trousers (4) and pair of socks (5).
